# FASCICULE DE BREVET EUROPEEN

(11) **EP 2 684 544 B1**
(45) Date de publication et mention de la délivrance du brevet: **21.01.2015**
(21) Numéro de dépôt: 13175368.3
(22) Date de dépôt: 05.07.2013
(51) Int. Cl.: A61F 2/46, A61B 17/17

(54) **Instrumentation chirurgicale spécifique à un patient pour la préparation du genou du patient**
Patientenspezifische chirurgische Instrumente für die Vorbereitung des Knies des Patienten
Patient-specific surgical instrument for preparing the knee of the patient

(30) Priorité: 09.07.2012 FR 1256601
(43) Date de publication de la demande: 15.01.2014
(73) Titulaire: Tornier, 38330 Montbonnot-Saint-Martin (FR)
(72) Inventeur: Zakaria, Toufik, 38100 GRENOBLE (FR); Bonnin, Michel, 69002 LYON (FR); Deschamps, Gérard, 71640 GIVRY (FR); Neyret, Philippe, 69890 La Tour de Salvagny (FR); Dejour, David, 69006 LYON (FR); Anract, Philippe, 78110 LE VESINET (FR)
(74) Mandataire: Grand, Guillaume

(56) Documents cités:
- WO-A1-2007/036699
- WO-A2-2012/024306
- US-A1- 2009 087 276
- US-A1- 2010 212 138

## Description

La présente invention concerne une instrumentation chirurgicale spécifique à un patient permettant de préparer le genou de ce patient, typiquement en vue d'y implanter une prothèse totale de genou.

Ainsi, l'invention porte sur une instrumentation que l'on qualifie également de « sur mesure » ou personnalisée, en lien avec un patient précis, exclusivement sur lequel l'instrumentation est destinée à être utilisée. Ce genre d'instrumentation spécifique à un patient s'oppose aux instrumentations standards, qui sont utilisées indifféremment sur divers patients, le cas échéant en étant réutilisées plusieurs fois de manière successive, en étant nettoyées et stérilisées entre chaque utilisation.

L'avènement des instrumentations « sur mesure » est lié aux possibilités actuelles d'acquérir des données préopératoires suffisamment précises afin de concevoir, notamment du point de vue dimensionnel, des instruments dont les interfaces de coopération mécanique avec les os du patient sont spécifiquement définies en tenant compte de la forme précise, notamment des reliefs de surface, de ces os. Les données préopératoires utilisées proviennent typiquement d'images scanner ou, plus généralement, de tout enregistrement de données de cartographie osseuse avantageusement obtenues de manière non invasive. Ces données sont traitées par ordinateur afin de commander la fabrication d'instruments chirurgicaux sur mesure, une fois que le chirurgien a décidé des détails de la procédure chirurgicale qu'il va suivre pas à pas lors d'une intervention à venir.

Dans ce contexte, l'invention s'intéresse plus spécifiquement aux instrumentations chirurgicales sur mesure destinées à préparer l'extrémité inférieure du fémur et/ou l'extrémité supérieure du tibia d'un patient, typiquement aux fins d'implantation des composants fémoral et tibial d'une prothèse de genou, étant remarqué que cette dernière peut aussi bien être une prothèse également sur mesure, c'est-à-dire personnalisée spécifiquement au patient à opérer, qu'une prothèse de genou « de catalogue », c'est-à-dire une prothèse standard, produite en série, le cas échéant déclinée en gamme dimensionnelle. Dans le contexte de la préparation de l'extrémité inférieure du fémur et/ou de l'extrémité supérieure du tibia, le recours à une instrumentation spécifique au patient à opérer présente un réel intérêt, du fait de la complexité de l'articulation du genou et de la nécessité de préparer les os du fémur et du tibia avec la plus grande précision possible, dans le sens où cette préparation détermine directement et significativement le positionnement d'implantation des composants fémoral et tibial sur le fémur et le tibia : on comprend donc que les performances mécaniques ultérieures de la prothèse implantée, et donc son usure et sa durée de vie, sont directement liées à la meilleure implantation possible, en ce qui concerne le positionnement des composants prothétiques vis-à-vis du fémur et du tibia.

En pratique, dans le contexte évoqué juste ci-dessus, les instrumentations sur mesure actuelles consistent généralement en un bloc monolithique, qui, comme expliqué plus haut, a été fabriqué en utilisant des données de cartographie osseuse préopératoires, relatives à un patient précis à opérer, et que le chirurgien utilise spécifiquement sur ce patient : ainsi, si on prend l'exemple du cas d'un bloc sur mesure pour la préparation du fémur, le chirurgien met en place ce bloc sur l'extrémité inférieure du fémur, selon une configuration unique prédéterminée, liée à la coopération par complémentarité de formes entre une surface d'appui fixe, délimitée par ce bloc, et l'extrémité inférieure du fémur, puis le chirurgien utilise ce guide pour contrôler l'application d'un ou de plusieurs outils de préparation osseuse, tels qu'un foret de perçage ou une broche d'ancrage. Ces outils permettent alors au chirurgien de préparer l'extrémité du fémur, notamment lui permettent de réséquer cette extrémité du fémur selon un ou plusieurs plans géométriques précis, ces plans de coupe étant notamment dimensionnés pour former des appuis plans correspondants pour la fixation d'un composant prothétique fémoral. Par nature, un tel bloc de guidage, spécifique au patient à opérer, ne laisse au chirurgien aucune possibilité d'ajustement quant à l'application des outils de préparation osseuse précitée : en effet, le recours à un tel bloc de guidage sur mesure vise, justement, à faciliter et sécuriser les gestes chirurgicaux, ces derniers étant réalisés, au cours de l'intervention, en suivant un planning opératoire prédéterminé par le chirurgien, notamment sur la base des données de cartographie osseuse préopératoires. En théorie, cette approche contrainte, à sens unique, garantit un résultat implantatoire optimal. Toutefois, dans la pratique, les chirurgiens constatent fréquemment que, en raison notamment de l'environnement ligamentaire de l'articulation du genou, le planning opératoire prédécidé peut ne pas être totalement satisfaisant en ce qui concerne le positionnement d'implantation relatif entre les composants prothétiques fémoral et tibial, eu égard à ce qui est couramment appelé la balance ligamentaire du genou, c'est-à-dire la résultante de tension entre la tension du ligament latéral interne et celle du ligament latéral externe du genou, ainsi que celles des ligaments croisés : en cas de déséquilibre de cette balance ligamentaire, une laxité résiduelle, positive ou négative, affecte l'un des compartiments interne et externe du genou par rapport à l'autre, avec des risques d'instabilité de la prothèse de genou implantée.

Pour contourner cette difficulté, il est recommandé au chirurgien utilisant des blocs de guidage sur mesure de vérifier la balance ligamentaire, mais ce après avoir reséqué l'extrémité du fémur comme expliqué plus haut : cependant, lorsque cette vérification s'avère négative, c'est-à-dire que le chirurgien constate un déséquilibre de la balance ligamentaire, le rééquilibrage de la balance ligamentaire oblige le chirurgien à tenter de corriger les coupes déjà réalisées, ce qui est particulièrement difficile, voire quasi-impossible notamment en cas de capital osseux insuffisant.

Cela explique pourquoi certains chirurgiens préfèrent continuer d'utiliser des ancillaires non spécifiques, autrement dit des ancillaires standards, dont la mise en oeuvre est certes plus longue et moins sécurisante que les instrumentations sur mesure, mais qui permettent, avant toute coupe du fémur, à la fois de contrôler et, si besoin, de rétablir l'équilibre de la balance ligamentaire et de mesurer l'écartement fémoro-tibial alors que le genou est dans une première configuration de pleine extension ou de flexion à 90°, puis, après avoir passé le genou dans l'autre configuration, de reporter cet écartement entre le fémur et le tibia en tenant compte de la balance ligamentaire et de contrôler de nouveau l'équilibre de la balance ligamentaire : de cette façon, en fonction de la valeur de l'écartement fémoro-tibial, les coupes de l'extrémité du fémur, notamment les coupes distales et postérieures, peuvent être positionnées de manière précise par rapport au tibia, notamment par rapport à une coupe préalable de l'extrémité supérieure du tibia, tout en garantissant l'équilibre de la balance ligamentaire du genou prothésé.

WO-A-2012/024306, sur lequel est basé le préambule de la revendication 1 annexée, a récemment proposé une instrumentation spécifique à un patient pour la préparation du genou du patient, dans laquelle un organe d'espacement est interposé entre l'extrémité réséquée du tibia et une partie dédiée d'un bloc fémoral spécifique au patient. Cet organe d'espacement coopère en contact plan-plan avec la partie dédiée précitée pour permettre au chirurgien de contrôler la balance ligamentaire du genou, et ce lorsque le genou est en complète extension, comme montré sur la figure 18 de WO-A-2012/024306, ou bien lorsque le genou est fléchi. Eu égard à la géométrie sensiblement plane de l'interface de contact entre cet organe d'espacement et le bloc fémoral spécifique au patient, on comprend que l'utilisation de cette instrumentation dans deux configurations de flexion-extension différentes du genou oblige le chirurgien à nécessairement dégager au moins la partie de l'instrumentation, se trouvant entre le fémur et le tibia, pendant que le genou est passé d'une première des deux configurations de flexion-extension à la seconde, avant que le chirurgien remette en place la totalité de l'instrumentation pour contrôler la balance ligamentaire dans la seconde configuration de flexion-extension.

Le but de la présente invention est de proposer une instrumentation sur mesure pour la préparation du genou, qui permet au chirurgien de contrôler facilement et rapidement la balance ligamentaire du genou dans plusieurs configurations de flexion-extension du genou.

A cet effet, l'invention a pour objet une instrumentation chirurgicale spécifique à un patient pour la préparation du genou du patient, telle que définie à la revendication 1.

Une des idées à la base de l'invention est de chercher à pouvoir contrôler l'équilibre de la balance ligamentaire du genou à l'aide d'un organe d'espacement entre le tibia et le fémur, à interposer entre un bloc spécifique au patient, fixé à un premier os, non encore réséqué, parmi le fémur et le tibia, et le second os, déjà réséqué. Afin d'être facile à manipuler et de fournir une donnée d'écartement fémoro-tibial pratique à exploiter pour le chirurgien, cet organe d'espacement délimite une surface incurvée qui est conçue pour coopérer avec une surface libre incurvée, délimitée par le bloc spécifique au patient : selon l'invention, lorsque, en utilisation, ces deux surfaces incurvées se retrouvent appuyées l'une contre l'autre de manière au moins sensiblement, voire rigoureusement ajustée, cela traduit l'équilibre de la balance ligamentaire pour la configuration de flexion-extension considérée du genou, l'équilibre de cette balance ligamentaire pouvant être ainsi vérifié continument sur toute une plage de flexion-extension du genou, et ce en laissant en place l'instrumentation. En pratique, eu égard à la façon dont est réalisé le bloc spécifique au patient, comme expliqué dans la partie introductive de ce document, on comprend que la surface libre précitée est ménagée sur le bloc spécifique au patient de manière précise et peut ainsi servir de référence positionnelle fiable alors que l'extrémité du premier os n'est pas encore réséquée. Bien entendu, une fois que l'équilibre de la balance ligamentaire est contrôlé à l'aide de l'organe d'espacement, le bloc spécifique au patient est avantageusement utilisé par le chirurgien pour poursuivre la préparation osseuse du premier os.

Ainsi, est également proposée ici une méthode de préparation du genou d'un patient, dans laquelle :
- sur une extrémité non réséquée d'un premier os parmi le fémur et le tibia du patient, tournée vers le second os, on appuie et fixe un bloc spécifique au patient, qui est conformé de manière spécifiquement ajustée à ladite extrémité du premier os,
- entre le bloc spécifique au patient, fixé sur ladite extrémité du premier os, et une extrémité réséquée du second os, tournée vers le premier os, on interpose un organe d'espacement, et
- on contrôle la balance ligamentaire du genou en vérifiant que, pendant que le genou est passé d'une configuration de flexion-extension à une autre configuration de flexion-extension, des surfaces incurvées, respectivement délimitées par le bloc spécifique au patient et par l'organe d'espacement, sont appuyées l'une contre l'autre de manière sensiblement ajustée.

Des caractéristiques additionnelles avantageuses de l'instrumentation conforme à l'invention sont spécifiées aux revendications dépendantes.

L'invention sera mieux comprise à la lecture de la description qui va suivre, donnée uniquement à titre d'exemple et faite en se référant aux dessins sur lesquels :
- la figure 1 est une vue en élévation d'un genou à préparer, montrant l'extrémité inférieure d'un fémur et l'extrémité supérieure d'un tibia associé ;
- la figure 2 est une vue en élévation similaire à la figure 1, montrant un bloc spécifique au patient appartenant à une instrumentation conforme à l'invention, en cours d'utilisation ;
- la figure 3 est une vue en élévation similaire à la figure 2, selon une direction opposée à cette dernière ;
- la figure 4 est une vue en perspective d'un organe d'espacement appartenant à l'instrumentation ;
- la figure 5 est une vue en perspective montrant le bloc spécifique au patient dans une configuration subséquente à celle des figures 2 et 3 et associé à l'organe d'espacement de la figure 4 ;
- la figure 6 est une vue similaire à la figure 2, illustrant le bloc spécifique au patient et l'organe d'espacement dans la configuration d'utilisation de la figure 5 ;
- la figure 7 est une vue en élévation suivant la flèche VII de la figure 6 ; et
- les figures 8 et 9 sont des coupes selon respectivement la ligne VIII-VIII de la figure 6 et la ligne IX-IX de la figure 7.

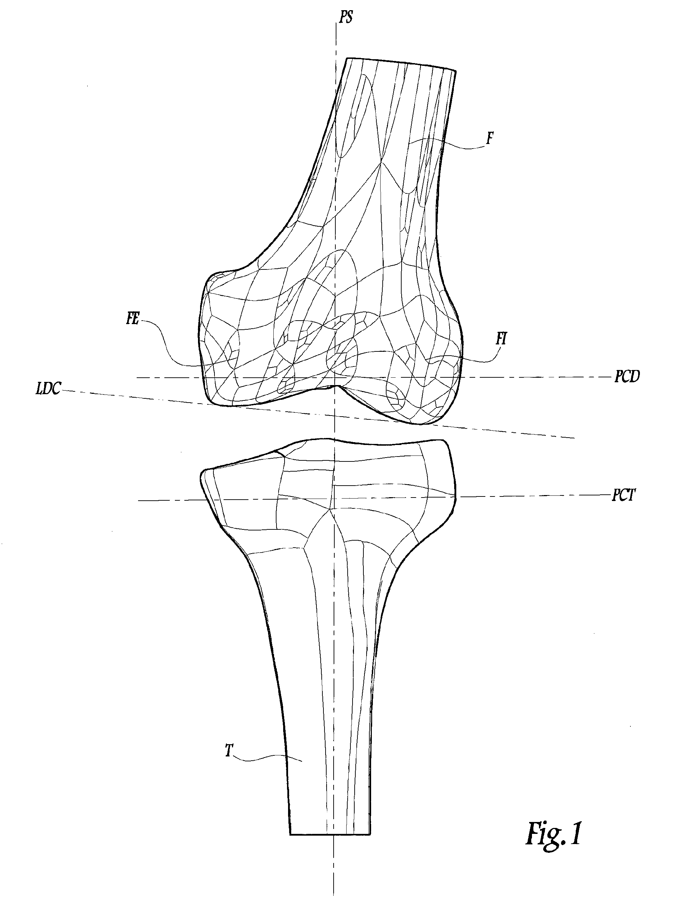

Sur la figure 1 sont représentées l'extrémité inférieure du fémur F et l'extrémité supérieure du tibia T d'un patient humain, qui sont tournées l'une vers l'autre et qui forment en partie le genou du patient. Sur la figure 1, ce genou est représenté en pleine extension, dans le sens anatomique du terme, c'est-à-dire avec les os du fémur F et du tibia T qui s'étendent dans le prolongement longitudinal l'un de l'autre.

Dans toute la suite de la description et, plus généralement, dans tout le présent document, les termes « supérieur », « inférieur », « vertical », « postérieur », etc. s'entendent dans leur sens anatomique usuel, en considérant que le patient opéré se tient debout sur une surface horizontale. Ainsi, la figure 1 correspond à une vue frontale du genou en pleine extension.

Pour des raisons qui apparaîtront plus loin, on a représenté sur la figure 1 plusieurs lignes en traits mixtes, à savoir :
- une ligne référencée PS qui correspond à la trace, dans le plan frontal, du plan sagittal du genou,
- une ligne référencée PCD qui correspond à la trace, dans le plan frontal, du plan d'une coupe distale à venir de l'extrémité inférieure du fémur F,
- une ligne référencée PCT qui correspond à la trace, dans le plan frontal, du plan d'une coupe horizontale à venir de l'extrémité supérieure du tibia T, les plans PCD et PCT étant sensiblement parallèles lorsque le genou est en pleine extension et la balance ligamentaire du genou est équilibrée, et
- une ligne référencée LDC qui est une droite géométrique appartenant au plan frontal et passant par, à la fois, le sommet distal du condyle interne FI du fémur F et le sommet distal du condyle externe FE du fémur.

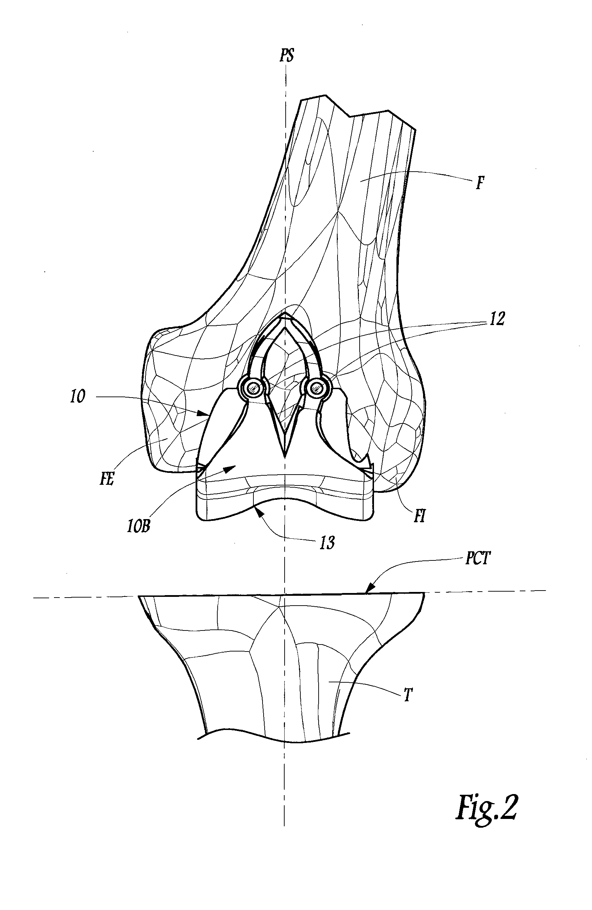

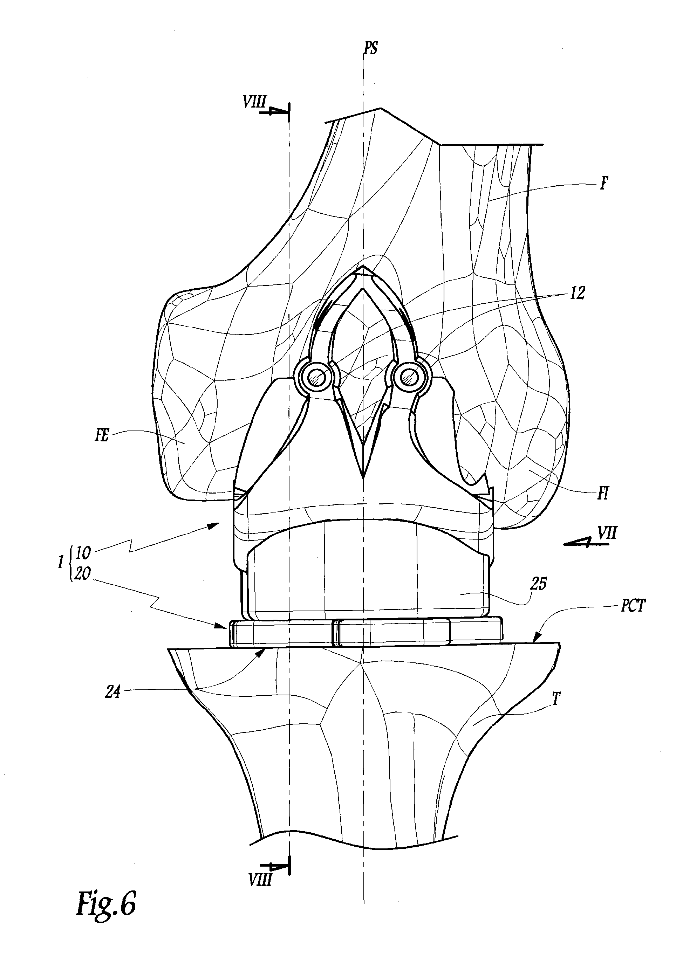

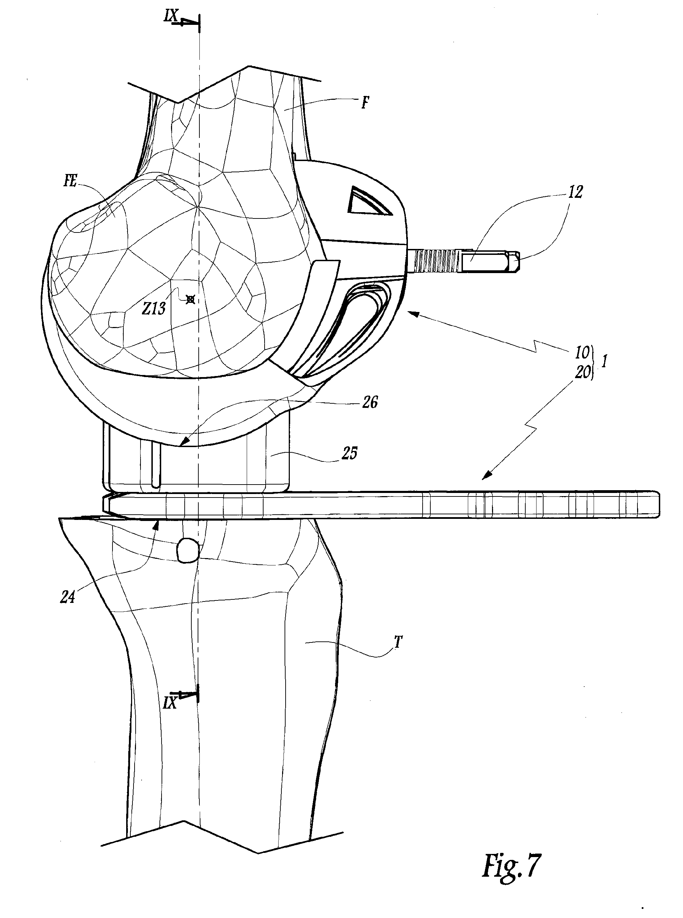

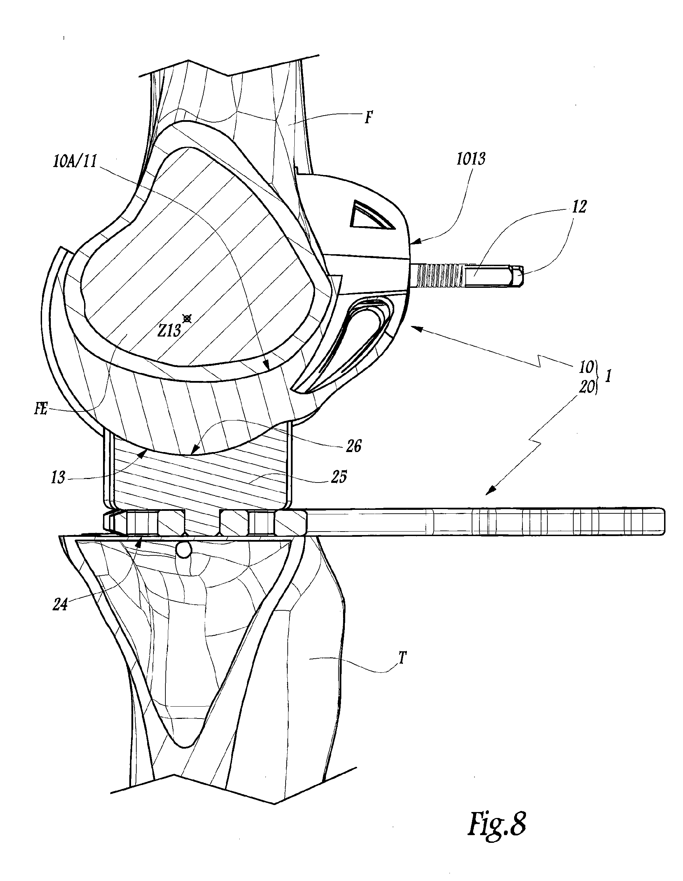

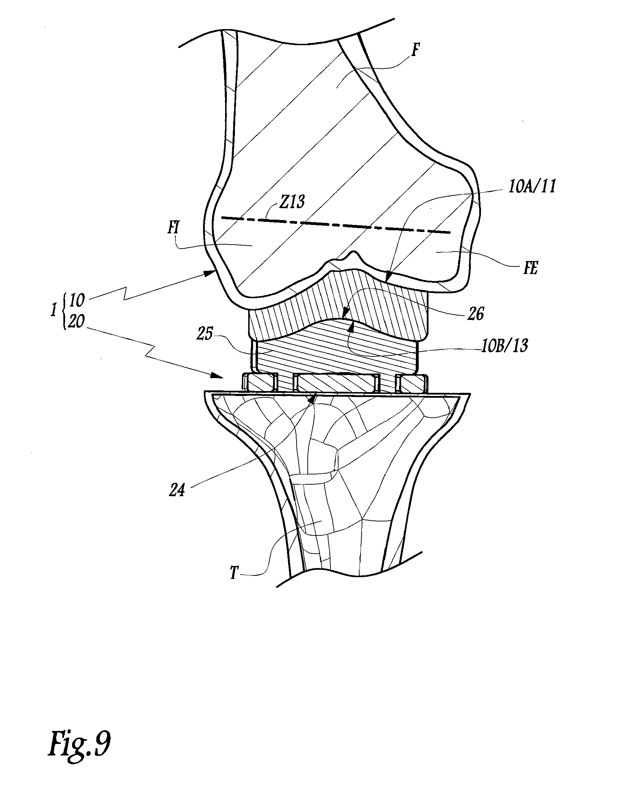

Sur les figures 2 à 9 est représentée une représentation chirurgicale 1 comprenant plusieurs composants qui vont être détaillés les uns après les autres, au fur et à mesure de la description d'une utilisation de cette instrumentation sur le genou de la figure 1, typiquement pour la préparation des extrémités en regard du fémur F et du tibia T à l'implantation des composants, fémoral et tibial, d'une prothèse de genou non représentée.

Préalablement à l'intervention chirurgicale d'implantation proprement dite, on recueille des données de cartographie relatives au genou du patient à opérer, en particulier relatives au fémur F. En pratique, ces données de cartographie préopératoires peuvent être obtenues de diverses manières. A titre d'exemple, des images scanner et/ou radiographiques et/ou échographiques et/ou d'IRM sont utilisées.

Dans tous les cas, à l'issue de cette étape préalable d'acquisition des données, on dispose de suffisamment d'informations pour concevoir et fabriquer un bloc fémoral 10 spécifique au patient, montré en position sur le fémur sur les figures 2, 3 et 5 à 9. Plus précisément, le bloc fémoral 10 présente, sur sa face 10A tournée en service vers le fémur F, une surface 11 qui est conformée de manière spécifiquement ajustée à l'extrémité inférieure du fémur F : en service, cette surface 11 est appuyée fixement contre cette extrémité de fémur, en épousant la surface de cette dernière par complémentarité de formes. On comprend que, pour aboutir à un tel ajustement rigoureux entre la surface d'appui 11 et l'extrémité inférieure du fémur F alors que cette dernière est dans son état naturel, en particulier sans avoir été préalablement réséquée ou, plus généralement, préparée par enlèvement ou remodelage de sa matière osseuse, la surface 11 est dessinée en utilisant les données de cartographie préopératoires relatives au fémur. De la sorte, la surface d'appui 11 présente des reliefs personnalisés spécifiques, qui, en coopérant avec des reliefs complémentaires délimités par la surface de l'extrémité inférieure du fémur, n'autorisent qu'une seule configuration d'appui ajusté sur le fémur F, comme représenté sur les figures 2, 3 et 5 à 9.

On notera que, dans le mode de réalisation considéré sur les figures, la surface d'appui 11 recouvre des zones de l'extrémité inférieure du fémur, qui sont situées de part et d'autre du plan sagittal PS, en épousant de manière ajustée les reliefs de ces zones. En particulier, la surface d'appui 11 recouvre des régions antérieures et distales du condyle interne FI, ainsi que des régions antérieures et distales du condyle externe FE, si bien que les sommets distaux de ces condyles, reliés par la ligne LDC, sont recouverts de manière ajustée par la surface 11. Ainsi, en d'autres termes, la surface d'appui 11 s'étend ici de part et d'autre du plan sagittal PS du genou de manière à coopérer avec les condyles interne et externe de l'extrémité du fémur F.

En service, le bloc fémoral 10 est destiné à être appuyé fixement, par sa surface 11, sur l'extrémité inférieure du fémur F. Dans l'exemple de réalisation considéré, cette fixation est réalisée grâce à deux broches d'ancrage osseux 12 conçues pour être respectivement engagées de façon complémentaire dans des trous traversants qui relient chacun la face 10A du bloc fémoral à sa face opposée 10B, c'est-à-dire celle tournée vers le chirurgien. Les broches d'ancrage 12 sont ainsi engagées dans les trous traversants précités jusqu'à se planter et ainsi s'immobiliser dans la matière osseuse du fémur F.

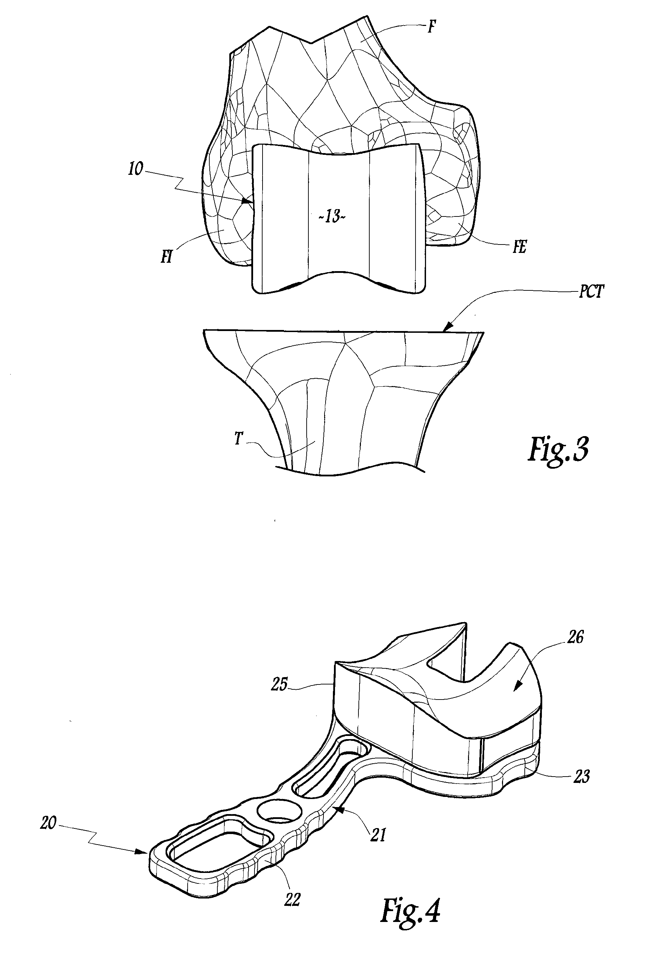

Sur sa face 10B, le bloc fémoral 10 délimite une surface incurvée 13, bien visible sur les figures 2 et 3. Dans le mode de réalisation considéré ici, cette surface incurvée 13 s'étend de part et d'autre du plan sagittal PS.

Dans l'exemple de réalisation considéré sur les figures, cette surface incurvée 13 s'apparente grossièrement à une portion de surface cylindrique dont l'axe central Z13 s'étend de manière médio-latérale, étant remarqué que cette portion de surface cylindrique présente un étranglement médian, ce qui rend la surface 13 similaire à une portion de diabolo. Plus généralement, pour des raisons qui apparaîtront un peu plus loin, la surface incurvée 13 est prévue pour présenter, en tout plan perpendiculaire à l'axe Z13, un profil sensiblement en arc de cercle centré sur cet axe.

En revenant maintenant à la description de l'intervention chirurgicale au cours de laquelle l'instrumentation 1 est utilisée, une première étape de cette intervention consiste, après avoir dégagé les chairs molles entourant l'extrémité inférieure du fémur et l'extrémité supérieure du tibia, à réséquer l'extrémité supérieure du tibia T : ainsi, lorsqu'on passe de la figure 1 aux figures 2 et 3, on observe que l'extrémité du tibia a été réséquée selon le plan de coupe tibial PCT. Pour ce faire, diverses possibilités, bien connues, sont à la disposition du chirurgien et ne sont pas limitatives de la présente invention.

Dans un deuxième temps opératoire, le chirurgien fixe le bloc fémoral 10 sur l'extrémité inférieure du fémur F, moyennant l'appui ajusté de sa surface 11 sur cette extrémité inférieure du fémur comme expliqué en détail plus haut. Après mise en place des broches d'ancrage 12, le bloc fémoral 10 est tel que représenté sur les figures 2 et 3, étant remarqué que, grâce à la résection préalable de l'extrémité supérieure du tibia T, un espace libre suffisant entre le fémur et le tibia reçoit, sans interférence, la partie du bloc fémoral 10 agencée en saillie de la région distale de l'extrémité inférieure du fémur F, même en pleine extension du genou.

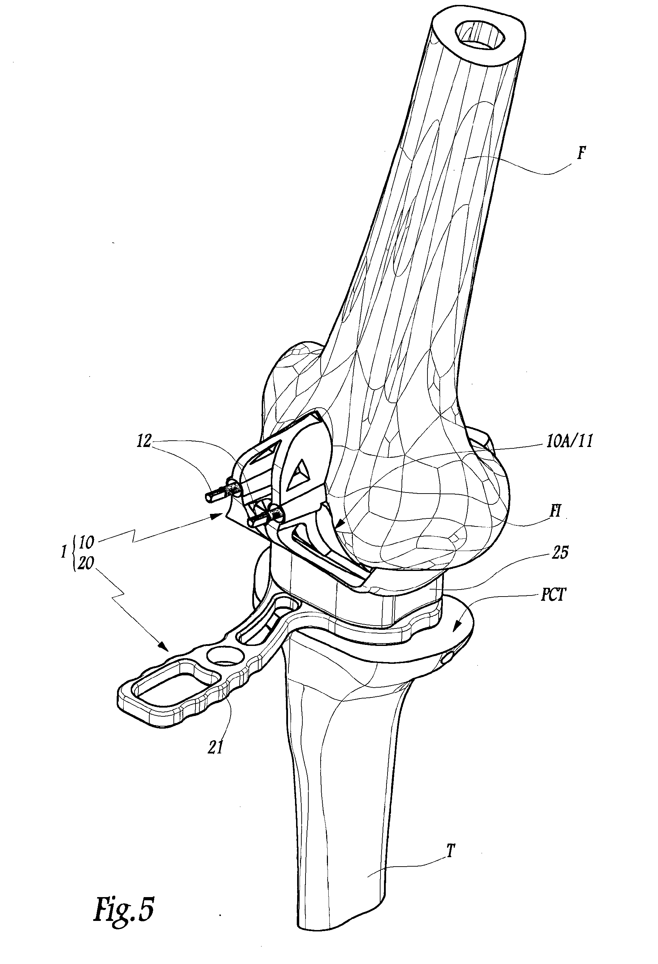

Dans un troisième temps opératoire qui est illustré par les figures 5 à 9, le bloc fémoral 10 est maintenu en place sur l'extrémité inférieure du fémur F, tandis que le chirurgien vient interposer, entre le bloc fémoral 10 et l'extrémité supérieure réséquée du tibia T, un organe d'espacement 20 appartenant à l'instrumentation 1. Cet organe d'espacement 20, qui est montré seul à la figure 4, comporte une palette de préhension 21 qui, à l'extrémité de sa poignée 22, inclut une plaquette 23. En service, la face inférieure de cette plaquette 23, c'est-à-dire sa face dirigée vers l'extrémité réséquée du tibia T, présente une surface plane 24 à même d'être appuyée en contact plan contre la résection de l'extrémité du tibia T. Sur sa face opposée, la plaquette 23 porte fixement un insert massif 25. Cet insert 25 délimite, sur sa face opposée à celle fixée à la palette 23, une surface incurvée 26, qui est bien visible sur la figure 21 et qui, comme visible sur les figures 17 et 18, est géométriquement complémentaire de la surface incurvée 13.

En service, lorsque l'organe d'espacement 20 est interposé entre l'extrémité supérieure réséquée du tibia T et le bloc fémoral 10 fixé à l'extrémité inférieure du fémur F, les surfaces incurvées 13 et 26 sont prévues pour coopérer de sorte que leur mise en appui de manière ajustée l'une contre l'autre caractérise l'équilibre de la balance ligamentaire du genou pour la configuration de flexion-extension de ce dernier considérée, typiquement dans la configuration de pleine extension sur les figures 5 à 9.

Si cette balance ligamentaire du genou en extension n'était pas équilibrée, les surfaces planes 13 et 26 ne seraient pas en appui jointif, mais, au contraire, s'écarteraient l'une de l'autre sur un des côtés interne et externe du genou. Bien entendu, si le chirurgien constate un défaut d'ajustement pour l'appui des surfaces 13 et 26 l'une contre l'autre, le chirurgien réalise des actes de correction de la balance ligamentaire, typiquement en relâchant la tension du ligament latéral interne ou celle du ligament latéral externe, jusqu'à faire sensiblement disparaître toute laxité résiduelle, ce qui s'observe par la fermeture du jour, externe ou interne, qui subsistait jusqu'alors entre les surfaces 13 et 26.

Eu égard au profil en arc de cercle des surfaces complémentaires 13 et 26, on comprend que la configuration de flexion-extension du genou peut être continument modifiée entre sa configuration de pleine extension et sa configuration de flexion à 90°, voire au-delà de cette dernière, tout en maintenant l'appui ajusté entre les surfaces 13 et 26 lorsque la balance ligamentaire du genou ainsi entraînée reste équilibrée.

Avantageusement, la forme en diabolo évoquée plus haut stabilise, suivant une direction médio-latérale, la coopération en rotation, autour de l'axe Z13, entre les surfaces 13 et 26.

Bien entendu, à titre de variante, la plage angulaire de flexion-extension du genou, pouvant être balayée pour vérifier l'équilibre de la balance ligamentaire du genou, peut être moins étendue que celle envisagée ci-dessus en regard des figures, moyennant un dimensionnement adéquat des surfaces incurvées 13 et 26 autour de leur axe de rotation relative Z13.

Par ailleurs, on comprend que le chirurgien a la possibilité de remplacer l'insert 25 utilisé par un insert ayant la même forme géométrique d'ensemble, à la différence que l'insert de remplacement présente une épaisseur différente de celle de l'insert précédemment utilisé lorsque le chirurgien constate que, avec l'insert précédemment utilisé, l'espace libre entre le bloc fémoral 10 fixé au fémur et l'extrémité réséquée du tibia est trop étroit pour y insérer l'organe d'espacement, ce qui traduit une trop forte épaisseur de l'insert précédemment utilisé, ou bien, au contraire, lorsque le chirurgien constate que, après mise en place de l'organe d'espacement 20, la totalité de la surface 26 reste distante de la totalité de la surface 13, ce qui traduit une trop faible épaisseur de l'insert précédemment utilisé. Sur la base des considérations qui précèdent, on comprend que, en fonction de l'insert 25 finalement retenu par le chirurgien pour lui permettre de contrôler l'équilibre de la balance ligamentaire, le chirurgien dispose d'une donnée dimensionnelle quant à l'écartement entre, d'une part, l'extrémité réséquée du tibia T et, d'autre part, le bloc fémoral 10 fixé au fémur F et donc, plus globalement, l'extrémité inférieure du fémur F eu égard au positionnement prédéterminé précis du bloc fémoral 10 sur le fémur F comme expliqué plus haut. Cette donnée dimensionnelle s'avère utile pour choisir le dimensionnement d'au moins certains des composant prothétiques de la prothèse de genou à implanter, ainsi que pour, le cas échéant, ajuster l'altitude du plan PCD et/ou abaisser l'altitude du plan PCT.

La suite de l'intervention chirurgicale, visant à implanter une prothèse de genou, ne sera pas détaillée dans la mesure où cet aspect n'est pas limitatif de la présente invention. On notera uniquement que, au cours de cette suite de l'intervention, l'extrémité inférieure du fémur F va être réséquée selon un plan de coupe distale, correspondant au plan PCD initialement envisagé ou à un plan parallèle situé à une hauteur différente par rapport au fémur, étant étendu que le plan dans lequel la coupe distale sera effectivement réalisée peut avantageusement être positionné avec précision grâce au bloc fémoral 10, par exemple en utilisant les broches d'ancrage 12 pour rapporter un bloc de coupe après avoir dégagé le bloc fémoral 10.

Ainsi, grâce à l'instrumentation 1, le chirurgien peut, sur toute une plage continue de flexion-extension du genou, contrôler efficacement l'équilibre de la balance ligamentaire. En particulier, comme expliqué plus haut, la surface incurvée 26 ne coopère pas directement avec l'extrémité inférieure du fémur F, ce qui, dans le cas contraire, rendrait difficile le contrôle de l'équilibre interne-externe de la balance ligamentaire en raison de l'inclinaison non orthogonale de la ligne LDC par rapport au plan sagittal PS, mais cette surface plane 26 est en mesure de coopérer avec une surface géométrique de référence définie par la surface 13 du bloc fémoral 10 conçue en conséquence. Bien entendu, cela est obtenu sans que le chirurgien n'ait encore réséqué la région distale de l'extrémité inférieure du fémur, ce qui lui laisse, en fonction des conséquences potentielles liées au rééquilibrage de la balance ligamentaire du genou, la possibilité de modifier le long du fémur la hauteur à laquelle sera réalisée la coupe distale selon le plan PCD.

Plus généralement, on comprend que la congruence rigoureuse des surfaces 13 et 26 permet de contrôler avec précision la balance ligamentaire dans toutes les configurations de flexion-extension entre les deux configurations extrêmes opposées précitées, en vérifiant l'espace entre le fémur et le tibia. A titre de variante, plutôt que d'être rigoureusement congruentes, les surfaces 13 et 26 peuvent être prédéterminées de manière à simuler l'interaction et la cinématique entre les composants d'une prothèse de genou à implanter : dans ce cas, ces surfaces s'articulent l'une sur l'autre lors de la flexion du genou, en s'appuyant l'une contre l'autre de manière sensiblement mais non rigoureusement ajustée, et ce de manière suffisante pour permettre un contrôle satisfaisant de la balance ligamentaire, tout en permettant de vérifier la cinématique de coopération entre ces surfaces.

Par ailleurs, dans les deux modes de réalisation évoqués juste ci-dessus, un aménagement optionnel consiste en ce que des moyens de mesure, tels que des capteurs de pression, des accéléromètres, des gyromètres, etc, sont intégrés à l'instrumentation pour caractériser et/ou quantifier la balance ligamentaire et/ou la cinématique de coopération entre les surfaces 13 et 26.

Divers aménagements et variantes à l'instrumentation 1 décrite jusqu'ici sont par ailleurs envisageables. A titre d'exemples :
- en variante non représentée, l'instrumentation peut être prévue unicompartimentale, c'est-à-dire que le bloc fémoral et l'organe d'espacement sont alors dimensionnés pour n'être utilisés qu'au niveau d'un seul des compartiments interne et externe du genou, étant entendu que, dans ce cas, cette instrumentation est particulièrement adaptée à la préparation du genou d'un patient en vue d'y implanter une prothèse totale unicompartimentale ;
- plutôt que de prévoir un bloc spécifique au patient à fixer au fémur, une solution « miroir » consiste à prévoir un bloc spécifique au patient à fixer à l'extrémité supérieure non réséquée du tibia T, autrement dit un bloc tibial spécifique au patient, étant entendu que, dans ce cas, d'une part, l'utilisation de l'instrumentation nécessite de commencer par une résection de l'extrémité inférieure du fémur et, d'autre part, cette instrumentation comprend un organe d'espacement à interposer entre l'extrémité inférieure réséquée du fémur et le bloc tibial précité, fixé à l'extrémité supérieure du tibia ; et/ou
- plutôt que d'être réalisé sous forme monolithique, comme c'est le cas pour le bloc fémoral 10 considéré sur les figures, le bloc spécifique au patient appartenant à l'instrumentation conforme à l'invention peut être réalisé en deux matériaux différents, en particulier pour ce qui concerne le corps principal de ce bloc spécifique au patient, tel que le corps principal 15 pour le bloc fémoral 10 ; ainsi, le bloc spécifique au patient inclut une première partie réalisée en un premier matériau, qui porte la surface d'appui fixe sur le fémur ou le tibia, ce premier matériau étant typiquement une matière plastique pour des raisons de facilité de fabrication de cette première partie ; et le bloc spécifique au patient inclut en outre une seconde partie solidaire de la première partie, qui porte la ou les surfaces libres, telles que la surface 13 de l'instrumentation 1, et qui est réalisée en un autre matériau que celui de la première partie, en prévoyant que cet autre matériau permet un bon glissement à l'interface de contact entre la ou les surfaces libres précitées et la surface complémentaire de l'organe d'espacement ; une telle option de fabrication trouve un intérêt particulier pour le mode de réalisation de l'instrumentation 1 en raison de la mobilité rotative entre le bloc 10 et l'organe d'espacement 20.

## Revendications

1. Instrumentation chirurgicale (1) spécifique à un patient pour la préparation du genou du patient, comportant :
- un bloc spécifique au patient (10), qui présente deux faces opposées délimitant respectivement :
une surface (11) d'appui fixe sur un premier os parmi le fémur (F) et le tibia (T) du patient, laquelle surface d'appui fixe est conformée de manière spécifiquement ajustée à une extrémité non réséquée du premier os, tournée vers le second os, de manière à coopérer avec au moins un des compartiments interne et externe de l'extrémité du premier os, et
une surface libre (13), et
- un organe d'espacement (20), qui est conçu pour, en service, être interposé de manière amovible entre le second os et le bloc spécifique au patient (10) fixé au premier os, et qui présente deux faces opposées délimitant respectivement :
une surface (24) d'appui sur une extrémité réséquée du second os, tournée vers le premier os, et
une surface (26) de coopération avec la surface libre (13) du bloc spécifique au patient (10),
**caractérisée en ce que** la surface libre (13) du bloc spécifique au patient (10) est incurvée de manière que cette surface libre et la surface de coopération (26) de l'organe d'espacement (20) sont adaptées pour s'appuyer de manière sensiblement ajustée l'une contre l'autre lorsque la balance ligamentaire du genou est équilibrée pendant que le genou est passé d'une configuration de flexion-extension à une autre configuration de flexion-extension.

2. Instrumentation suivant la revendication 1, **caractérisée en ce que** la surface libre (13) du bloc spécifique au patient (10) et la surface de coopération (26) de l'organe d'espacement (20) sont adaptées pour s'appuyer de manière ajustée l'une contre l'autre lorsque la balance ligamentaire du genou est équilibrée pendant que le genou est passé au moins de sa configuration de pleine extension à sa configuration de flexion à 90°.

3. Instrumentation suivant l'une des revendications 1 ou 2, **caractérisée en ce que** la surface libre incurvée (13) du bloc spécifique au patient (10) présente, en tout plan perpendiculaire à un axe géométrique médio-latérale, un profil sensiblement en arc de cercle, centré sur cet axe.

4. Instrumentation suivant l'une quelconque des revendications précédentes, **caractérisée en ce que** la surface libre (13) du bloc spécifique au patient (10) et la surface de coopération (26) de l'organe d'espacement (20) sont rigoureusement congruentes.

5. Instrumentation suivant l'une des revendications 1 à 3, **caractérisée en ce que** la surface libre du bloc spécifique au patient et la surface de coopération de l'organe d'espacement sont adaptées pour s'articuler l'une contre l'autre de manière non complètement congruente, notamment pour simuler une cinématique de prothèse du genou.

6. Instrumentation suivant l'une quelconque des revendications précédentes, **caractérisée en ce que** la surface d'appui fixe (11) du bloc spécifique au patient (10) est dimensionnée pour s'étendre de part et d'autre d'un plan sagittal (PS) du genou.

7. Instrumentation suivant l'une quelconque des revendications précédentes, **caractérisée en ce que** le bloc spécifique au patient (10) est conçu pour être fixé au fémur (F).

8. Instrumentation suivant l'une quelconque des revendications précédentes, **caractérisée en ce que** le bloc spécifique au patient (10) inclut à la fois :
- une première partie, qui porte la surface d'appui fixe et qui est réalisée en un premier matériau, notamment une matière plastique, et
- une seconde partie, qui porte la ou les surfaces libres, qui est solidarisée fixement à la première partie et qui est réalisée en un second matériau, différent du premier matériau et formant un contact glissant avec la surface de coopération de l'organe d'espacement.

## Patentansprüche

1. Patientenspezifisches Instrument für die Vorbereitung des Knies des Patienten, umfassend:
- einen patientenspezifischen Block (10); der zwei entgegengesetzte Flächen aufweist, die jeweils begrenzen:
eine Fläche (11) zur festen Auflage auf einem ersten Knochen, ausgewählt aus dem Femur (F) und dem Tibia (T) des Patienten, wobei die Fläche zur festen Auflage in einer solchen Art ausgebildet ist,
dass sie an ein nicht reseziertes, zu dem zweiten Knochen gerichtetes Ende des ersten Knochens angepasst ist, so dass sie mit mindestens einem von dem inneren und dem äußeren Kompartiment des Endes des ersten Knochens zusammenarbeitet, und
eine freie Fläche (13), und
- ein Abstandselement (20), das konzipiert ist, im Betrieb in lösbarer Weise zwischen dem zweiten Knochen und dem an dem ersten Knochen befestigten patientenspezifischen Block (10) angeordnet zu werden, und das zwei entgegengesetzte Flächen aufweist, die jeweils begrenzen:
eine zu dem ersten Knochen gerichtete Fläche (24) zur Auflage auf ein reseziertes Ende des zweiten Knochens, und
eine Fläche (26) zum Zusammenwirken mit der freien Fläche (13) des patientenspezifischen Blocks (10),
**dadurch gekennzeichnet, dass** die freie Fläche (13) des patientenspezifischen Blocks (10) so gekrümmt ist, dass diese freie Fläche und die Fläche (26) zum Zusammenwirken des Abstandselements (20) angepasst sind, sich in im Wesentlichen angepasster Weise gegeneinander abzustützen, wenn die Weichteil-Balancierung des Knies ausgeglichen ist, während das Knie von einer Beugungs-Streckungs-Konfiguration in eine andere Beugungs-Streckungs-Konfiguration übergegangen ist.

2. Instrument nach Anspruch 1, **dadurch gekennzeichnet, dass** die freie Fläche (13) des patientenspezifischen Blocks (10) und die Fläche (26) zum Zusammenwirken des Abstandselements (20) angepasst sind, sich in angepasster Weise gegeneinander abzustützen, wenn die Weichteil-Balancierung des Knies ausgeglichen ist, während das Knie mindestens von seiner Konfiguration der vollständigen Streckung in seine Konfiguration einer 90° Beugung übergegangen ist.

3. Instrument nach einem der Ansprüche 1 oder 2, **dadurch gekennzeichnet, dass** die gekrümmte freie Fläche (13) des patientenspezifischen Blocks (10) in jeder Ebene senkrecht zu einer medial-lateralen geometrischen Achse ein im Wesentlichen kreisbogenförmiges, auf diese Achse zentriertes Profil aufweist.

4. Instrument nach einem beliebigen der vorhergehenden Ansprüche,
**dadurch gekennzeichnet, dass** die freie Fläche (13) des patientenspezifischen Blocks (10) und die Fläche (26) zum Zusammenwirken des Abstandselements (20) streng kongruent sind.

5. Instrument nach einem der Ansprüche 1 bis 3, **dadurch gekennzeichnet, dass** die freie Fläche des patientenspezifischen Blocks und die Fläche zum Zusammenwirken des Abstandselements angepasst sind, eine Gelenkverbindung in nicht vollständig kongruenter Weise zueinander zu bilden, insbesondere um eine Kinematik der Knieprothese zu simulieren.

6. Instrument nach einem beliebigen der vorhergehenden Ansprüche, **dadurch gekennzeichnet, dass** die Fläche (11) zur festen Auflage des patientenspezifischen Blocks (10) so dimensioniert ist, dass sie sich beidseitig einer Sagittalebene (PS) des Knies erstreckt.

7. Instrument nach einem beliebigen der vorhergehenden Ansprüche, **dadurch gekennzeichnet, dass** der patientenspezifische Block (10) ausgebildet ist, an dem Femur (F) befestigt zu werden.

8. Instrument nach einem beliebigen der vorhergehenden Ansprüche, **dadurch gekennzeichnet, dass** der patientenspezifische Block (10) gleichzeitig einschließt:
- einen ersten Bereich, der die Fläche zur festen Auflage trägt und der aus einem ersten Material, insbesondere Kunststoff, hergestellt ist, und
- einen zweiten Bereich, der die freie(n) Fläche(n) trägt, der fest mit dem ersten Bereich verbunden ist und der aus einem zweiten Material, unterschiedlich zum ersten Material hergestellt ist und einen gleitenden Kontakt mit der Fläche zum Zusammenwirken des Abstandselements bildet.

## Claims

1. Patient-specific surgical instrumentation (1) for preparing the knee of the patient comprising:
• a patient-specific block (10) that has two opposing faces respectively delimiting:
a fixed support surface (11) on a first bone, either the femur (F) or the tibia (T) of the patient, wherein the fixed support surface is specifically shaped to fit a non-resected end of the first bone and is turned towards the second bone in order to cooperate with at least one of the internal and external compartments of the end of the first bone, and
a free surface (13), and
• a spacer element (20), which is designed to be detachably interposed, when in use, between the second bone and the patient-specific block (10) fixed to the first bone and which has two opposing faces respectively delimiting:
a fixed support surface (24) on a resected end of the second bone and turned towards the first bone, and
a surface (26) for cooperating with the free surface (13) of the patient-specific block (10),
**characterised in that** the free surface (13) of the patient-specific block (10) is curved in such a manner that this free surface and the cooperating surface (26) of the spacer element (20) are arranged to rest against one another in a substantially fitted manner when the ligaments of the knee are balanced while the knee moves from one flexion-extension configuration to another flexion-extension configuration.

2. Instrumentation according to claim 1, **characterised in that** the free surface (13) of the patient-specific block (10) and the cooperating surface (26) of the spacer element (20) are arranged to rest against one another in a fitted manner when the ligaments of the knee are balanced while the knee moves at least from its configuration of full extension to its configuration of flexion to 90°.

3. Instrumentation according to one of claims 1 or 2, **characterised in that** in any plane perpendicular to a mediolateral geometric axis, the curved free surface (13) of the patient-specific block (10) has a substantially circular arc-shaped profile centred on this axis.

4. Instrumentation according to any one of the preceding claims, **characterised in that** the free surface (13) of the patient-specific block (10) and the cooperating surface (26) of the spacer element (20) are strictly congruent.

5. Instrumentation according to one of claims 1 to 3, **characterised in that** the free surface of the patient-specific block and the cooperating surface of the spacer element are arranged to be articulated one against the other in a manner that is not completely congruent, in particular to simulate kinematics of the knee prosthesis.

6. Instrumentation according to any one of the preceding claims, **characterised in that** the fixed supporting surface (11) of the patient-specific block (10) is dimensioned so as to extend either side of a sagittal plane (PS) of the knee.

7. Instrumentation according to any one of the preceding claims, **characterised in that** the patient-specific block (10) is designed to be fixed to the femur (F).

8. Instrumentation according to any one of the preceding claims, **characterised in that** the patient-specific block (10) includes both:
• a first part, which carries the fixed supporting surface and which is made from a first material, in particular a plastic material, and
• a second part, which carries the free surface or surfaces, is fixedly joined to the first part and is made from a second material different from the first material, forming a sliding contact with the cooperating surface of the spacer element.
